(19) Europäisches Patentamt
European Patent Office
Office européen des brevets

(11) EP 1 057 471 B1

(12) **FASCICULE DE BREVET EUROPEEN**

(45) Date de publication et mention de la délivrance du brevet:
**11.05.2005 Bulletin 2005/19**

(51) Int Cl.[7]: **A61K 7/13**

(21) Numéro de dépôt: **00401362.9**

(22) Date de dépôt: **18.05.2000**

(54) **Composition de teinture d'oxydation des fibres kératiniques et procédé de teinture mettant en oeuvre cette composition**

Oxidationsfärbungszusammensetzung für keratinische Fasern und Färbungsverfahren mit derselben

Composition for oxidative dying of keratinous fibres and dying process therewith

(84) Etats contractants désignés:
**AT BE CH CY DE DK ES FI FR GB GR IE IT LI LU MC NL PT SE**

(30) Priorité: **04.06.1999 FR 9907092**

(43) Date de publication de la demande:
**06.12.2000 Bulletin 2000/49**

(73) Titulaire: **L'OREAL**
**75008 Paris (FR)**

(72) Inventeurs:
- **Plos, Grégory**
**75015 Paris (FR)**
- **Kravtchenko, Sylvain**
**75012 Paris (FR)**

(74) Mandataire: **Miszputen, Laurent**
**L'OREAL - D.I.P.I.**
**25-29 Quai Aulagnier**
**92600 Asnières (FR)**

(56) Documents cités:
**WO-A-00/32158**
**WO-A-97/41215**
**DE-A- 19 713 852**
**GB-A- 1 320 250**
**WO-A-94/00100**
**WO-A-99/15137**
**FR-A- 2 750 048**
**US-A- 5 899 212**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

L'invention a pour objet une composition pour la teinture d'oxydation des fibres kératiniques, et en particulier des fibres kératiniques humaines telles que les cheveux comprenant, dans un milieu approprié pour la teinture, au moins une base d'oxydation, au moins une enzyme de type oxydo-réductase ammoniogène à 2 électrons, et au moins un donneur pour ladite enzyme, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu fortement alcalin, en présence de peroxyde d'hydrogène. Toutefois, l'utilisation des milieux alcalins en présence de peroxyde d'hydrogène présente pour inconvénient d'entraîner une dégradation non négligeable des fibres, ainsi qu'une décoloration des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi il a déjà été proposé de teindre les fibres kératiniques, notamment dans la demande de brevet EP-A-0 310 675, avec des compositions comprenant un précurseur de colorant d'oxydation de type benzénique, en association avec des enzymes telles que la pyranose-oxydase, la glucose-oxydase ou bien l'uricase, en présence d'un donneur pour lesdites enzymes. Dans la demande de brevet WO 941 00100, il est décrit un procédé pour la teinture d'oxydation des fibres kératiniques consistant à appliquer sur ces fibres un premier composant comprenant au moins un dérivé indolique ou indolinique, un deuxième composant comprenant au moins une enzyme de type peroxydase et un troisième composant comprenant au moins du peroxyde d'hydrogène ou une source de peroxyde d'hydrogène enzymatique, le premier et le troisième composants étant appliqués séparément. Ces procédés de teinture, bien qu'étant mis en oeuvre dans des conditions n'entraînant pas une dégradation des fibres kératiniques comparable à celle engendrée par les teintures réalisées en présence de peroxyde d'hydrogène, ne sont pas entièrement satisfaisantes, notamment en ce qui concerne la puissance des colorations obtenues. Se pose aussi parfois le problème du manque de solubilité du donneur, ce qui est notamment le cas de l'acide urique qui est le donneur correspondant à l'uricase.

Or, la demanderesse vient maintenant de découvrir qu'il est possible d'obtenir de nouvelles teintures, capables de conduire à des colorations plus puissantes que celles de l'art antérieur mettant en oeuvre un système enzymatique, en associant au moins une base d'oxydation, au moins une enzyme de type oxydoréductase ammoniogène à 2 électrons, et au moins un donneur pour ladite enzyme.

Cette découverte est à la base de la présente invention.

L'invention a donc pour premier objet une composition prête à l'emploi, pour la teinture d'oxydation des fibres kératiniques et en particulier des fibres kératiniques humaines telles que les cheveux, caractérisée par le fait qu'elle comprend, dans un milieu approprié pour la teinture :

- au moins une base d'oxydation,
- au moins une enzyme de type oxydo-réductase ammoniogène à 2 électrons,
- et au moins un donneur pour ladite enzyme.

La composition tinctoriale conforme à l'invention permet d'obtenir des colorations plus puissantes que celles obtenues avec les compositions de l'art antérieur utilisant un système oxydant enzymatique, tel que par exemple le système oxydant acide urique / uricase.

De plus, les colorations obtenues avec la composition tinctoriale conforme à l'invention sont peu sélectives et résistent bien aux différentes agressions que peuvent subir les cheveux, (lumière, intempéries, lavages, déformations permanentes, etc...).

L'invention a également pour objet un procédé de teinture d'oxydation des fibres kératiniques mettant en oeuvre cette composition tinctoriale prête à l'emploi.

Selon l'invention, le terme ammoniogène signifie : qui libère de l'ammoniac ou de l'ammoniaque au cours de la réaction enzyme-donneur.

La nature de la ou des bases d'oxydation utilisées dans la composition tinctoriale prête à l'emploi n'est pas critique. Elles peuvent notamment être choisies parmi les paraphénylènediamines, les bases doubles, les para-aminophénols, les ortho aminophénols et les bases d'oxydation hétérocycliques.

Parmi les paraphénylènediamines utilisables à titre de base d'oxydation dans les composition tinctoriales conformes à l'invention, on peut notamment citer les composés de formule (I) suivante et leurs sels d'addition avec un acide :

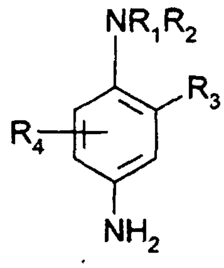

(I)

dans laquelle :

- $R_1$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), alkyle en $C_1$-$C_4$ substitué par un groupement azoté, phényle ou 4'-aminophényle ;
- $R_2$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$) ou alkyle en $C_1$-$C_4$ substitué par un groupement azoté ;
- $R_3$ représente un atome d'hydrogène, un atome d'halogène tel qu'un atome de chlore, de brome, d'iode ou de fluor, un radical alkyle en $C_1$-$C_4$, monohydroxyalkyle en $C_1$-$C_4$, hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$,
- $R_4$ représente un atome d'hydrogène, d'halogène ou un radical alkyle en $C_1$-$C_4$.

Parmi les groupements azotés de la formule (I) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les paraphénylènediamines de formule (I) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N, N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Selon l'invention, on entend par bases doubles, les composés comportant au moins deux noyaux aromatiques

sur lesquels sont portés des groupements amino et/ou hydroxyle.

Parmi les bases doubles utilisables à titre de bases d'oxydation dans les compositions tinctoriales conformes à l'invention, on peut notamment citer les composés de formule (II) suivante, et leurs sels d'addition avec un acide :

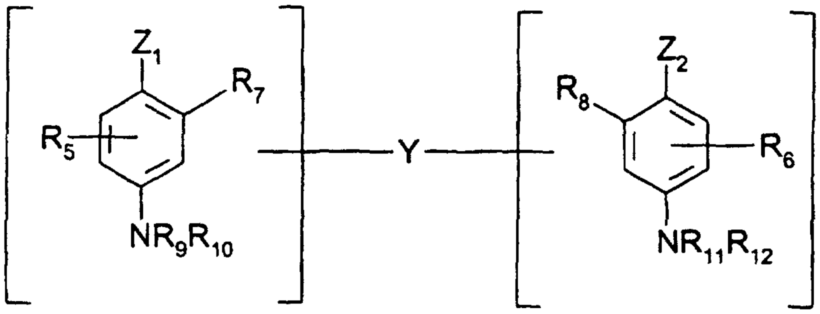

(II)

dans laquelle :

- $Z_1$ et $Z_2$, identiques ou différents, représentent un radical hydroxyle ou $-NH_2$ pouvant être substitué par un radical alkyle en $C_1$-$C_4$ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_5$ et $R_6$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_7$, $R_8$, $R_9$, $R_{10}$ $R_{11}$ et $R_{12}$, identiques ou différents, représentent un atome d'hydrogène, un bras de liaison Y ou un radical alkyle en $C_1$-$C_4$ ;

étant entendu que les composés de formule (II) ne comportent qu'un seul bras de liaison Y par molécule.

Parmi les groupements azotés de la formule (II) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les bases doubles de formules (II) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N, N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de bases d'oxydation dans les compositions tinctoriales conformes à l'invention, on peut notamment citer les composés de formule (III) suivante, et leurs sels d'addition avec un acide :

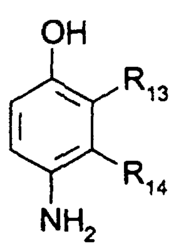

(III)

dans laquelle :

- $R_{13}$ représente un atome d'hydrogène ou d'halogène, un radical alkyle en $C_1$-$C_4$, monohydroxyalkyle en $C_1$-$C_4$, alcoxy($C_1$-$C_4$)alkyle($C_1$-$C_4$), aminoalkyle en $C_1$-$C_4$ ou hydroxyalkyl($C_1$-$C_4$)aminoalkyle en $C_1$-$C_4$,
- $R_{14}$ représente un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$),

étant entendu qu'au moins un des radicaux $R_{13}$ ou $R_{14}$ représente un atome d'hydrogène.

Parmi les para-aminophénols de formule (III) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation représentent de préférence de 0,0005 à 12 % en poids environ du poids total de la composition tinctoriale prête à l'emploi conforme à l'invention, et encore plus préférentiellement de 0,005 à 6 % en poids environ de ce poids.

Les enzymes de type oxydo-réductase ammoniogène à 2 électrons pouvant être utilisées dans la composition tinctoriale prête à l'emploi conforme à l'invention sont définies par la classe E.C. 1.4.3 de la nomenclature des enzymes, (voir Enzyme Nomenclature, Academic Press Inc., 1989).

Lesdites enzymes peuvent notamment être choisies parmi les D-aspartate oxydases (E.C. 1.4.3.1.), les L-amino-acide oxydases (E.C. 1.4.3.2.), les D-amino-acide oxydases (E.C. 1.4.3.3.), les (mono)amine oxydases contenant une flavine (E.C. 1.4.3.4.), les pyridoxaminephosphate oxydases (E.C. 1.4.3.5.), les (di)amine oxydases contenant du cuivre (E.C. 1.4.3.6.), les D-glutamate oxydases (E.C. 1.4.3.7), les éthanolamine oxydases (E.C. 1.4.3.8), les putrescine oxydases (E.C. 1.4.3.10), les L-glutamate oxydases (E.C. 1.4.3.11), les cyclohexylamine oxydases (E.C.

1.4.3.12), les protéine-lysine-6-oxydases (E.C. 1.4.3.13), les L-lysine oxydase (E.C. 1.4.3.14), les D-glutamate (D-aspartate) oxydases (E.C. 1.4.3.15), et les L-aspartate oxydases (E.C. 1.4.3.16).

Selon une forme de réalisation préférée de l'invention, la ou les enzymes de type oxydo-réductase ammoniogène à 2 électrons sont choisies parmi les sous-classes des amino-acide oxydase, c'est à dire parmi les classes E.C. 1.4.3.1, E.C. 1.4.3.2, E.C. 1.4.3.3, E.C. 1.4.3.7, E.C. 1.4.3.11, E.C. 1.4.3.14, E.C. 1.4.3.15 et E.C. 1.4.3.16.

La ou les oxydo-réductases ammoniogènes à 2 électrons utilisées dans la composition tinctoriale prête à l'emploi conforme à l'invention peut être d'origine animale, microbiologique (bactérienne, fongique ou virale) ou synthétique (obtenue par synthèse chimique ou biotechnologique).

La ou les oxydo-réductases ammoniogènes à 2 électrons utilisées dans la composition tinctoriale prête à l'emploi conforme à l'invention représentent de préférence de 0,01 à 40 % en poids environ du poids total de la composition tinctoriale prête à l'emploi, et encore plus préférentiellement de 0,1 à 10 % en poids environ de ce poids.

L'activité enzymatique des oxydo-réductases ammoniogènes à 2 électrons utilisées conformément à l'invention peut être définie à partir de l'oxydation du donneur en condition aérobie. Une unité U correspond à la quantité d'enzyme conduisant à la génération de 1 µmole de peroxyde d'hydrogène par minute à un pH de 8,5 et à une température de 25°C.

De façon préférentielle, la quantité d'oxydo-réductases ammoniogènes à 2 électrons (s) est comprise entre 10000 et $4.10^8$ unités U environ pour 100 g de composition tinctoriale.

Selon l'invention, on entend par donneur, le ou les différents substrats nécessaires au fonctionnement de l'oxydo-réductase ammoniogène à 2 électrons utilisée.

La nature du donneur (ou substrat) pour ladite enzyme varie en fonction de la nature de l'oxydo-réductase ammoniogène à 2 électrons qui est utilisée. Par exemple, à titre de donneur pour les D-aspartate oxydases, on peut citer le D-aspartate et le D-glutamate ; à titre de donneur pour les L-amino-acide oxydases, on peut citer la L-glycine, le L-alanine, la L-valine, la L-phénylalanine et le L-tryptophane ; à titre de donneur pour les D-amino-acide oxydases, on peut citer la D-alanine, et la D-phénylalanine ; à titre de donneur pour les (mono)amine oxydases contenant une flavine, on peut citer la benzylamine et l'octylamine ; à titre de donneur pour les pyridoxaminephosphate oxydases, on peut citer la pyridoxine phosphate et la pyridoxamine phosphate ; à titre de donneur pour les (di)amine oxydases contenant du cuivre, on peut citer le 1,4-diamino butane, le 1,4-diamino pentane et la spermidine ; à titre de donneur pour les D-glutamate oxydases, on peut citer le D-glutamate ; à titre de donneur pour les éthanolamines oxydases, on peut citer la monoéthanolamine, le 3-amino-1-propanol et le 1-amino-2-propanol ; à titre de donneur pour les putrescine oxydases, on peut citer la putrescine ; à titre de donneur pour les L-glutamate oxydases, on peut citer le L-glutamate ; à titre de donneur pour les cyclohexylamine oxydases, on peut citer la cyclohexylamine et les cycloamines ; à titre de donneur pour les protéine-lysine-6-oxydases, on peut citer le peptidyl-L-lysyl-peptide ; à titre de donneur pour les L-lysine oxydases, on peut citer la L-lysine, la L-ornithine, la L-histidine, la L-phénylalanine et la L-arginine ; à titre de donneur pour les D-glutamate (D-aspartate) oxydases, on peut citer le D-glutamate et le D-aspartate ; et enfin, à titre de donneur pour les L-aspartate oxydases, on peut citer le L-aspartate.

Le ou les donneurs (ou substrats) utilisés conformément à l'invention représentent de préférence de 0,01 à 20 % en poids environ du poids total de la composition tinctoriale prête à l'emploi conforme à l'invention et encore plus préférentiellement de 0,1 à 5 % en poids environ.

La composition tinctoriale prête à l'emploi conforme à l'invention peut également renfermer un ou plusieurs coupleurs et/ou un ou plusieurs colorants directs, notamment pour modifier les nuances ou les enrichir en reflets.

Parmi les coupleurs utilisables dans les compositions tinctoriales prêtes à l'emploi conformes à l'invention, on peut notamment citer les métaphénylènediamines, les méta-aminophénols, les métadiphénols et les coupleurs hétérocycliques tels que par exemple les dérivés indoliques, les dérivés indoliniques, les dérivés de benzimidazole, les dérivés de benzomorpholine, les dérivés de sésamol, les dérivés pyridiniques, pyrimidiniques et pyrazoliques, et leurs sels d'addition avec un acide.

Ces coupleurs sont plus particulièrement choisis parmi le 2-méthyl 5-amino phénol, le 5-N-(β-hydroxyéthyl) amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents le ou les coupleurs représentent de préférence de 0,0001 à 10 % en poids environ du poids total de la composition tinctoriale prête à l'emploi et encore plus préférentiellement de 0,005 à 5 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en $C_1$-$C_4$, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition prête à l'emploi conforme à l'invention est choisi de telle manière que l'activité enzymatique de l'oxydo-réductase ammoniogène à 2 électrons soit suffisante. Il est généralement compris entre 5 et 11 environ, et de préférence entre 6,5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (IV) suivante :

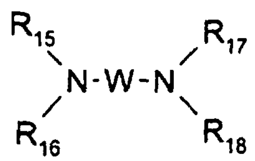

(IV)

dans laquelle W est un reste propylène éventuellement substitué par un groupement hydroxyle ou un radical alkyle en $C_1$-$C_4$ ; $R_{15}$, $R_{16}$, $R_{17}$ et $R_{18}$, identiques ou différents, représentent un atome d'hydrogène, un radical alkyle en $C_1$-$C_4$ ou hydroxyalkyle en $C_1$-$C_4$.

La composition tinctoriale prête à l'emploi conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des enzymes différentes de l'oxydo-réductase ammoniogène à 2 électrons utilisée conformément à l'invention telles que par exemples des peroxydases, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.

La composition tinctoriale prête à l'emploi conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains. Dans ce cas, le ou les colorants d'oxydation et la ou les oxydo-réductases ammoniogènes à 2 électrons sont présents au sein de la même composition prête à l'emploi, et par conséquent ladite composition doit être exempte d'oxygène gazeux, de manière à éviter toute oxydation prématurée du ou des colorants d'oxydation.

L'invention a également pour objet un procédé de teinture d'oxydation des fibres kératiniques et en particulier des fibres kératiniques humaines telles que les cheveux mettant en oeuvre la composition tinctoriale prête à l'emploi telle que définie précédemment.

Selon ce procédé, on applique sur lesdites fibres au moins une composition tinctoriale prête à l'emploi telle que définie précédemment, pendant un temps suffisant pour développer la coloration désirée, après quoi on rince, on lave éventuellement au shampooing, on rince à nouveau et on sèche.

Le temps nécessaire au développement de la coloration sur lesdites fibres kératiniques est généralement compris entre 3 et 60 minutes et encore plus précisément 5 et 40 minutes.

Selon une autre forme de réalisation particulière de l'invention, le procédé comporte une étape préliminaire consistant à stocker sous forme séparée, d'une part, une composition (A) comprenant, dans un milieu approprié pour la teinture, au moins une base d'oxydation et, d'autre part, une composition (B) renfermant, dans un milieu approprié pour la teinture, au moins une enzyme de type oxydo-réductase ammoniogène à 2 électrons, ladite composition (A) et/ou ladite composition (B) renfermant au moins un donneur pour ladite enzyme, puis à procéder à leur mélange au moment de l'emploi avant d'appliquer ce mélange sur les fibres kératiniques.
Les exemples qui suivent sont destinés à illustrer l'invention sans pour autant en limiter la portée.

## EXEMPLES

### EXEMPLES 1 et 2 DE TEINTURE

On a préparé les compositions tinctoriales prêtes à l'emploi conformes à l'invention suivantes :

| COMPOSITION | 1 | 2 |
|---|---|---|
| Paraphénylènediamine (base d'oxydation) | 0,324 g | - |
| Para-aminophénol (base d'oxydation) | - | 0,327 g |
| le 1-amino 2-méthoxy 4,5-méthylènedioxy benzène (coupleur) | 0,611 g | - |
| 2-méthyl 5-amino phénol (coupleur) | - | 0,369 g |
| D-alanine oxydase commercialisée par la société Sigma (référence A1614) (enzyme conforme à l'invention) | 20000 U | - |
| D-alanine oxydase commercialisée par la société Sigma (référence A1914) (enzyme conforme à l'invention) | - | 20000 U |
| D-alanine (donneur) | 0,535 g | 0,535 g |
| 2-amino-2-méthyl-1-propanol q.s. pH | 9 | 9 |
| Eau déminéralisée q.s.p. | 100 g | 100 g |

Chacune des compositions tinctoriales prêtes à l'emploi décrites ci-dessus a été appliquée sur des mèches de cheveux gris naturels à 90 % de blancs pendant 30 minutes à température ambiante. Les cheveux ont ensuite été rincés, lavés avec un shampooing standard, puis séchés.
Les cheveux ont été teints dans les nuances figurant dans le tableau ci-dessous :

| EXEMPLE | NUANCE OBTENUE |
|---|---|
| 1 | Doré mat cendré |
| 2 | Doré |

### EXEMPLES DE TEINTURE COMPARATIFS 3 ET 4

On a préparé les compositions tinctoriales prêtes à l'emploi suivantes :

| COMPOSITION | 3 | 4 (*) |
|---|---|---|
| Paraphénylènediamine (base d'oxydation) | 0,324 g | 0,324 g |
| 2-méthyl 5-amino phénol (coupleur) | 0,369 g | 0,369 g |
| D-alanine oxydase commercialisée par la société Sigma (référence A1614) (enzyme conforme à l'invention) | 20000 U | - |
| D-alanine (donneur) | $3.10^{-3}$ mole | - |
| Uricase d'Arthrobacter globiformis à 20 U.I / mg, commercialisée par la société Sigma | | 20000 U |
| Acide urique | | $3.10^{-3}$ mole |

(*) exemple comparatif ne faisant pas partie de l'invention

(suite)

| COMPOSITION | 3 | 4 (*) |
|---|---|---|
| 2-amino-2-méthyl-1-propanol q.s. pH | 9,75 ± 0,25 | 9,75 ± 0,25 |
| Eau déminéralisée q.s.p. | 100 g | 100 g |

(*) exemple comparatif ne faisant pas partie de l'invention

Chacune des compositions tinctoriales prêtes à l'emploi décrites ci-dessus a été appliquée sur des mèches de cheveux gris naturels à 90 % de blancs pendant 30 minutes à température ambiante. Les cheveux ont ensuite été rincés, lavés avec un shampooing standard, puis séchés.
La couleur des mèches a été évaluée avant et après la teinture, dans le système L* a* b*, au moyen d'un spectrophotomètre CM 2002 MINOLTA ®, (Illuminant D65).
Dans le système L* a* b*, les trois paramètres désignent respectivement l'intensité (L*), la nuance (a*) et la saturation (b*).
Selon ce système, plus la valeur de L est élevée, plus la couleur est claire ou peu intense. Inversement, plus la valeur de L est faible, plus la couleur est foncée ou très intense.
a* et b* indiquent deux axes de couleurs, a* indique l'axe de couleur vert/rouge et b* l'axe de couleur bleu/jaune. Des valeurs proches de zéro pour a* et b* correspondent à des nuances grises.
La montée de la coloration (ΔE) peut être calculée en appliquant l'équation suivante :

$$\Delta E = \sqrt{(L^* - L_0{}^*)^2 + (a^* - a_0{}^*)^2 + (b^* - b_0{}^*)^2}$$

Dans cette équation, ΔE représente la différence de couleur entre deux mèches, (dans le cas présent la montée de la coloration), L*, a*, et b* représentent respectivement l'intensité, la nuance et la saturation de la mèche teinte, $L_0$*, $a_0$* et $b_0$* représentant respectivement l'intensité, la nuance et la saturation de la mèche non teinte.
Plus la valeur de ΔE est importante, plus la différence de couleur entre les deux mèches est importante, et dans le cas présent, plus la montée de la coloration est importante.
Pour chacune des compositions testées, l'écart de coloration ΔE entre les mèches non teintes et les mèches teintes a été calculée selon la formule indiquée ci-dessus, de façon à pouvoir évaluer la puissance de la coloration.
Les résultats obtenus figurent dans le tableau ci-après :

| Exemple | Couleur de la mèche non teinte | | | Couleur de la mèche teinte | | | Puissance de la coloration (ΔE) |
|---|---|---|---|---|---|---|---|
| | L* | a* | b* | L* | a* | b* | |
| **3** | 54 | 1 | 12 | 27 | 13 | 3 | **31** |
| **4 (*)** | 54 | 1 | 12 | 31 | 13 | 4 | **26** |

Ces résultats montrent que la composition de l'exemple 3 conforme à l'invention, c'est à dire contenant de la D-alanine oxydase, qui est une oxydo-réductase ammoniogène à 2 électrons, en présence de D-alanine à titre de donneur, conduit à une coloration plus puissante que la composition de l'exemple 4 selon l'art antérieur contenant de l'uricase, qui n'est pas une oxydo-réductase ammoniogène à 2 électrons, et de l'acide urique à titre de donneur.

## Revendications

1. Composition prête à l'emploi, pour la teinture d'oxydation des fibres kératiniques et en particulier des fibres kératiniques humaines telles que les cheveux, **caractérisée par le fait qu'**elle comprend, dans un milieu approprié pour la teinture :

   - au moins une base d'oxydation,
   - au moins une enzyme de type oxydo-réductase ammonlogène à 2 électrons,
   - et au moins un donneur pour ladite enzyme.

2. Composition selon la revendication 1, **caractérisée par le fait que** les bases d'oxydation sont choisies parmi les

paraphénylènediamines, les bases doubles, les para-aminophénols, les ortho aminophénols et les bases d'oxydation hétérocycliques.

**3.** Composition selon la revendication 2, **caractérisée par le fait que** les bases d'oxydation sont choisies parmi les paraphénylènediamines, les bases doubles, les para-aminophénols, les orthoaminophénols.

**4.** Composition selon la revendication 2 ou 3, **caractérisée par le fait que** les paraphénylènediamines sont choisies parmi les composés de formule (I) suivante et leurs sels d'addition avec un acide :

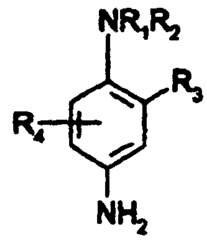

(I)

dans laquelle :

- $R_1$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), alkyle en $C_1$-$C_4$ substitué par un groupement azoté, phényle ou 4'-aminophényle ;
- $R_2$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$) ou alkyle en $C_1$-$C_4$ substitué par un groupement azoté ;
- $R_3$ représente un atome d'hydrogène, un atome d'halogène tel qu'un atome de chlore, de brome, d'iode ou de fluor, un radical alkyle en $C_1$-$C_4$, monohydroxyalkyle en $C_1$-$C_4$, hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$,
- $R_4$ représente un atome d'hydrogène, d'halogène ou un radical alkyle en $C_1$-$C_4$.

**5.** Composition selon la revendication 4, **caractérisée par le fait que** les paraphénylènediamines de formule (I) sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

**6.** Composition selon la revendication 2 or 3, **caractérisée par le fait que** les bases doubles sont choisies parmi les composés de formule (II) suivante, et leurs sels d'addition avec un acide :

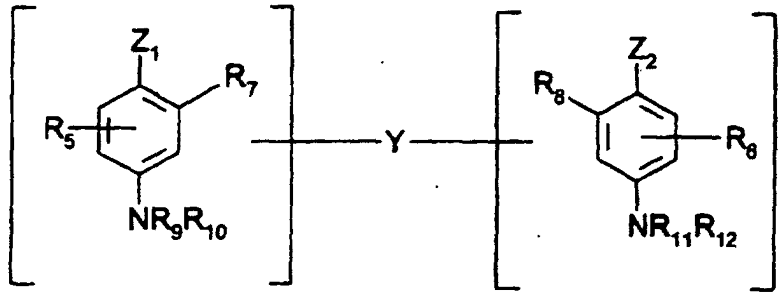

(II)

dans laquelle :

- $Z_1$ et $Z_2$, identiques ou différents, représentent un radical hydroxyle ou $-NH_2$ pouvant être substitué par un radical alkyle en $C_1$-$C_4$ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_5$ et $R_6$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_7$, $R_8$, $R_9$, $R_{10}$ $R_{11}$ et $R_{12}$, identiques ou différents, représentent un atome d'hydrogène, un bras de liaison Y ou un radical alkyle en $C_1$-$C_4$ ;

étant entendu que les composés de formule (II) ne comportent qu'un seul bras de liaison Y par molécule.

7. Composition selon la revendication 6, **caractérisée par le fait que** les bases doubles de formules (II) sont choisies parmi le N,N'-bis-(β-hydroxyéthyl) N,N'-bis-(4'-aminophényl) 1,3-diamino propanol, la N,N-bis-(β-hydroxyéthyl) N, N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

8. Composition selon la revendication 2 ou 3 **caractérisée par le fait que** les para-aminophénols sont choisis parmi les composés de formule (III) suivante, et leurs sels d'addition avec un acide :

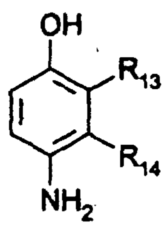

(III)

dans laquelle:

- $R_{13}$ représente un atome d'hydrogène ou d'halogène, un radical alkyle en $C_1$-$C_4$, monohydroxyalkyle en $C_1$-$C_4$, alcoxy($C_1$-$C_4$)alkyle($C_1$-$C_4$), aminoalkyle en $C_1$-$C_4$ ou hydroxyalkyl($C_1$-$C_4$)aminoalkyle en $C_1$-$C_4$,
- $R_{14}$ représente un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$),

étant entendu qu'au moins un des radicaux $R_{13}$ ou $R_{14}$ représente un atome d'hydrogène.

9. Composition selon la revendication 8, **caractérisée par le fait que** les para-aminophénols de formule (III) sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminomémyl phénol, le 4-amino 2-(β-hydroxyéthyl aminométhyl) phénol, le 4-amino 2-fluoro phénol, et leurs sels d'addition avec un acide.

10. Composition selon la revendication 2 ou 3, **caractérisée par le fait que** les orthoaminophénols sont choisis parmi 2-amino phénol, le 2-amino 5-méthyl phénol, le 2-amino 6-méthyl phénol, le 5-acétamido 2-amino phénol, et leurs sels d'addition avec un acide.

11. Composition selon la revendication 2, **caractérisée par le fait que** les bases hétérocycliques sont choisies parmi les dérivés pyridiniques, les dérivés pyrimidiniques, les dérivés pyrazoliques, et leurs sels d'addition avec un acide.

12. Composition selon l'une quelconque des revendications précédentes, **caractérisée par le fait que** la ou les bases

d'oxydation représentent de 0,0005 à 12 % en poids du poids total de la composition tinctoriale prête à l'emploi.

**13.** Composition selon l'une quelconque des revendications précédentes, **caractérisée par le fait que** les enzymes de type oxydo-réductase ammoniogène à 2 électrons sont choisies parmi les D-aspartate oxydases, les L-amino-acide oxydases, les D-amino-acide oxydases, les (mono)amine oxydases contenant une flavine, les pyridoxaminephosphate oxydases, les (di)amine oxydases contenant du cuivre, les D-glutamate oxydases, les éthanolamine oxydases, les putrescine oxydases, les L-glutamate oxydases, les cyclohexylamine oxydases, les protéine-lysine-6-oxydases, les L-lysine oxydase, les D-glutamate (D-aspartate) oxydases, et les L-aspartate oxydases.

**14.** Composition selon l'une quelconque des revendications précédentes, **caractérisée par le fait que** la ou les oxydo-réductases ammoniogènes à 2 électrons représentent de préférence de 0,01 à 40 % en poids du poids total de la composition tinctoriale prête à l'emploi.

**15.** Composition selon rune quelconque des revendications précédentes, **caractérisée par le fait que** le ou les donneurs représentent de 0,01 à 20 % en poids du poids total de la composition tinctoriale prête à l'emploi.

**16.** Composition selon l'une quelconque des revendications précédentes, **caractérisée par le fait qu'**elle renferme un ou plusieurs coupleurs et/ou un ou plusieurs colorants directs.

**17.** Composition selon la revendication 16, **caractérisée par le fait que** les coupleurs sont choisis parmi les métaphénylènediamines, les méta-aminophénols, les métadiphénols, les coupleurs hétérocycliques, et leurs sels d'addition avec un acide.

**18.** Composition selon la revendication 17, **caractérisée par le fait que** les coupleurs sont choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthyl-amino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

**19.** Composition selon l'une quelconque des revendications 16 à 18, **caractérisée par le fait que** le ou les coupleurs représentent de 0,0001 à 10 % en poids du poids total de la composition tinctoriale prête à l'emploi.

**20.** Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates.

**21.** Procédé de teinture d'oxydation des fibres kératiniques et en particulier des fibres kératiniques humaines telles que les cheveux, **caractérisé par le fait qu'**on applique sur lesdites fibres au moins une composition tinctoriale prête à l'emploi telle que définie à l'une quelconque des revendications 1 à 20, pendant un temps suffisant pour développer la coloration désirée.

**22.** Procédé selon la revendication 21, **caractérisé par le fait que** le temps nécessaire au développement de la coloration sur lesdites fibres kératiniques est compris entre 3 et 60 minutes.

**23.** Procédé selon la revendication 21 ou 22, **caractérisé par le fait qu'**il comporte une étape préliminaire consistant à stocker sous forme séparée, d'une part, une composition (A) comprenant, dans un milieu approprié pour la teinture, au moins une base d'oxydation, et d'autre part, une composition (B) renfermant, dans un milieu approprié pour la teinture, au moins une enzyme de type oxydo-réductase ammoniogène à 2 électrons, ladite composition (A) et/ou ladite composition (B) renfermant au moins un donneur pour ladite enzyme, puis à procéder à leur mélange au moment de l'emploi avant d'appliquer ce mélange sur les fibres kératiniques.

## Patentansprüche

**1.** Gebrauchsfertige Zusammensetzung zum oxidativen Färben von Keratinfasern und insbesondere menschlichen Keratinfasern, wie Haaren, **dadurch gekennzeichnet, dass** sie in einem zum Färben geeigneten Träger enthält:

- mindestens eine Oxidationsbase,
- mindestens ein Enzym vom Typ der ammoniogenen Oxidoreduktasen, die 2 Elektronen übertragen, und
- mindestens einen Donor für das Enzym.

**2.** Zusammensetzung nach Anspruch 1, **dadurch gekennzeichnet, dass** die Oxidationsbasen unter den p-Phenylendiaminen, Doppelbasen, p-Aminophenolen, o-Aminophenolen und den heterocyclischen Basen ausgewählt sind.

**3.** Zusammensetzung nach Anspruch 2, **dadurch gekennzeichnet, dass** die Oxidationsbasen unter den p-Phenylendiaminen, Doppelbasen, p-Aminophenolen und o-Aminophenolen ausgewählt sind.

**4.** Zusammensetzung nach Anspruch 2 oder 3, **dadurch gekennzeichnet, dass** die p-Phenylendiamine unter den Verbindungen der folgenden Formel (I) und deren Additionssalzen mit einer Säure ausgewählt sind:

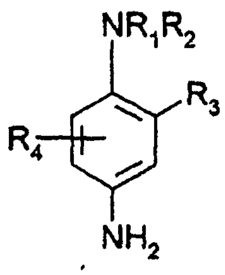 (I)

worin bedeuten:

- $R_1$ Wasserstoff, $C_{1-4}$-Alkyl, $C_{1-4}$-Monohydroxyalkyl, $C_{2-4}$-Polyhydroxyalkyl, $C_{1-4}$-Alkoxy-$C_{1-4}$-alkyl, eine mit einer stickstoffhaltigen Gruppe substituierte $C_{1-4}$-Alkylgruppe, Phenyl oder 4'-Aminophenyl,
- $R_2$ Wasserstoff, $C_{1-4}$-Alkyl, $C_{1-4}$-Monohydroxyalkyl, $C_{2-4}$-Polyhydroxyalkyl, $C_{1-4}$-Alkoxy-$C_{1-4}$-alkyl oder eine mit einer stickstoffhaltigen Gruppe substituierte $C_{1-4}$-Alkylgruppe,
- $R_3$ Wasserstoff, Halogen, wie Chlor, Brom, Iod oder Fluor, $C_{1-4}$-Alkyl, $C_{1-4}$-Monohydroxyalkyl, $C_{1-4}$-Hydroxyalkoxy, $C_{1-4}$-Acetylaminoalkoxy, $C_{1-4}$-Mesylaminoalkoxy oder $C_{1-4}$-Carbamoylaminoalkoxy, und
- $R_4$ Wasserstoff, Halogen oder $C_{1-4}$-Alkyl.

**5.** Zusammensetzung nach Anspruch 4, **dadurch gekennzeichnet, dass** die p-Phenylendiamine der Formel (I) unter p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-N,N-diethyl-3-methylanilin, N,N-Bis(β-hydroxyethyl)-p-phenylendiamin, 4-N,N-Bis(β-hydroxyethyl)amino-2-methyl-anilin, 4-N,N-Bis(β-hydroxyethyl)-amino-2-chlor-anilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-pphenylendiamin, N,N-Dimethyl-3-methyl-p-phenylendiamin, N,N-(Ethyl, β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-β-Hydroxyethyloxy-pphenylendiamin, 2-β-Acetylaminoethyloxy-p-phenylendiamin, N-(β-Methoxyethyl)-p-phenylendiamin und deren Additionssalzen mit einer Säure ausgewählt sind.

**6.** Zusammensetzung nach Anspruch 2 oder 3, **dadurch gekennzeichnet, dass** die Doppelbasen unter den Verbindungen der folgenden Formel (II) und deren Additionssalze mit einer Säure ausgewählt sind:

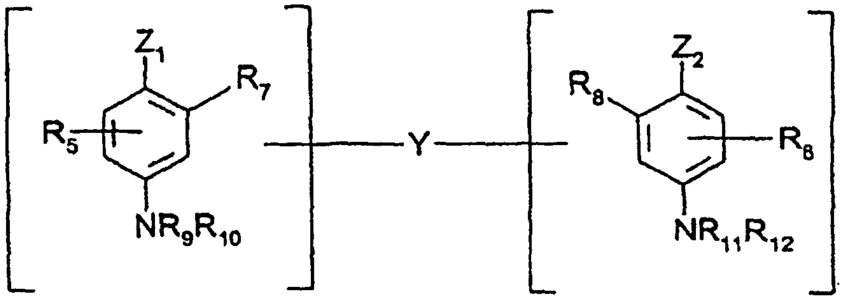 (II)

worin bedeuten:

- $Z_1$ und $Z_2$, die identisch oder voneinander verschieden sind, Hydroxy oder eine $NH_2$-Gruppe, die mit $C_{1-4}$-Alkyl oder einer Verbindungsgruppe Y substituiert sein kann,
- die Verbindungsgruppe Y eine geradkettige oder verzweigte Alkylengruppe mit 1 bis 14 Kohlenstoffatomen, die durch eine oder mehrere stickstoffhaltige Gruppen und/oder ein oder mehrere Heteroatome, wie Sauerstoff, Schwefel oder Stickstoff, unterbrochen oder abgeschlossen werden und gegebenenfalls mit einer oder mehreren Hydroxy- oder $C_{1-6}$-Alkoxygruppen substituiert sein kann,
- $R_5$ und $R_6$ Wasserstoff, Halogen, $C_{1-4}$-Alkyl, $C_{1-4}$-Monohydroxyalkyl, $C_{2-4}$-Polyhydroxyalkyl, $C_{1-4}$-Aminoalkyl oder eine Verbindungsgruppe Y,
- $R_7$, $R_8$, $R_9$, $R_{10}$, $R_{11}$ und $R_{12}$, die identisch oder voneinander verschieden sind, Wasserstoff, eine Verbindungsgruppe Y oder $C_{1-4}$-Alkyl,

mit der Maßgabe, dass die Verbindungen der Formel (II) nur eine Verbindungsgruppe Y pro Molekül aufweisen.

7. Zusammensetzung nach Anspruch 6, **dadurch gekennzeichnet, dass** die Doppelbasen der Formel (II) unter N, N'-Bis(β-hydroxyethyl)-N,N'-bis-(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis (4-aminophenyl)-tetramethylendiamin, N,N'-Bis(4-methylaminophenyl)-tetramethylendiamin, N,N'-Bis(ethyl)-N, N'-bis-(4'-amino-3'-methylphenyl)-ethylendiamin, 1,8-Bis(2,5-diamino-phenoxy)-3,5-dioxaoctan und deren Additionssalzen mit einer Säure ausgewählt sind.

8. Zusammensetzung nach Anspruch 2 oder 3, **dadurch gekennzeichnet, dass** die p-Aminophenole unter den Verbindungen der folgenden Formel (III) und deren Additionssalze mit einer Säure ausgewählt sind:

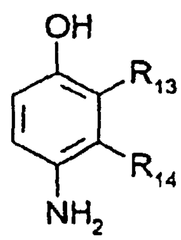 (III)

worin bedeuten:

- $R_{13}$ Wasserstoff, Halogen, $C_{1-4}$-Alkyl, $C_{1-4}$-Monohydroxyalkyl, $C_{1-4}$-Alkoxy-$C_{1-4}$-alkyl, $C_{1-4}$-Aminoalkyl oder $C_{1-4}$-Hydroxyalkyl-$C_{1-4}$-aminoalkyl,
- $R_{14}$ Wasserstoff, Halogen, $C_{1-4}$-Alkyl, $C_{1-4}$-Monohydroxyalkyl, $C_{2-4}$-Polyhydroxyalkyl, $C_{1-4}$-Aminoalkyl, $C_{1-4}$-Cyanoalkyl oder $C_{1-4}$-Alkoxy-$C_{1-4}$-alkyl,

mit der Maßgabe, dass mindestens eine der Gruppen $R_{13}$ oder $R_{14}$ Wasserstoff bedeutet.

**9.** Zusammensetzung nach Anspruch 8, **dadurch gekennzeichnet, dass** die p-Aminophenole der Formel (III) unter p-Aminophenol, 4-Amino-3-methyl-phenol, 4-Amino-3-fluor-phenol, 4-Amino-3-hydroxymethyl-phenol, 4-Amino-2-methyl-phenol, 4-Amino-2-hydroxymethyl-phenol, 4-Amino-2-methoxymethyl-phenol, 4-Amino-2-aminomethyl-phenol, 4-Amino-2-(β-hydroxyethylaminomethyl)-phenol, 4-Amino-2-fluor-phenol und deren Additionssalzen mit einer Säure ausgewählt sind.

**10.** Zusammensetzung nach Anspruch 2 oder 3, **dadurch gekennzeichnet, dass** die o-Aminophenole unter 2-Aminophenol, 2-Amino-5-methyl-phenol, 2-Amino-6-methyl-phenol, 5-Acetamido-2-amino-phenol und deren Additionssalzen mit einer Säure ausgewählt sind.

**11.** Zusammensetzung nach Anspruch 2, **dadurch gekennzeichnet, dass** die heterocyclischen Basen unter den Pyridinderivaten, Pyrimidinderivaten, Pyrazolderivaten und deren Additionssalzen mit einer Säure ausgewählt sind.

**12.** Zusammensetzung nach einem der vorhergehenden Ansprüche, **dadurch gekennzeichnet, dass** die Oxidationsbase(n) 0,0005 bis 12 Gew.-% des Gesamtgewichts der gebrauchsfertigen Farbmittelzusammensetzung ausmachen.

**13.** Zusammensetzung nach einem der vorhergehenden Ansprüche, **dadurch gekennzeichnet, dass** die Enzyme vom Typ der ammoniogenen Oxidoreductasen, die 2 Elektronen übertragen, unter den D-Aspartat-Oxidasen, L-Aminosäure-Oxidasen, D-Aminosäure-Oxidasen, (Mono)amin-Oxidasen, die ein Flavin enthalten, Pyridoxaminphosphat-Oxidasen, (Di)amin-Oxidasen, die Kupfer enthalten, D-Glutamat-Oxidasen, Ethanolamin-Oxidasen, Putrescin-Oxidasen, L-Glutamat-Oxidasen, Cyclohexylamin-Oxidasen, Protein-Lysin-6-Oxidasen, L-Lysin-Oxidasen, D-Glutamat(D-Aspartat)-Oxidasen und L-Aspartat-Oxidasen ausgewählt werden.

**14.** Zusammensetzung nach einem der vorhergehenden Ansprüche, **dadurch gekennzeichnet, dass** die ammoniogenen Oxidoreduktase(n) (2 Elektronen) vorzugsweise 0,01 bis 40 Gew.-% des Gesamtgewichts der gebrauchsfertigen Farbmittelzusammensetzung ausmachen.

**15.** Zusammensetzung nach einem der vorhergehenden Ansprüche, **dadurch gekennzeichnet, dass** der Donor oder die Donoren 0,01 bis 20 Gew.-% des Gesamtgewichts der gebrauchsfertigen Farbmittelzusammensetzung ausmachen.

**16.** Zusammensetzung nach einem der vorhergehenden Ansprüche, **dadurch gekennzeichnet, dass** sie ferner einen oder mehrere Kuppler und/oder einen oder mehrere Direktfarbstoffe enthält.

**17.** Zusammensetzung nach Anspruch 16, **dadurch gekennzeichnet, dass** die Kuppler unter den m-Phenylendiaminen, m-Aminophenolen, m-Dihydroxybenzolen und den heterocyclischen Kupplern und deren Additionssalzen mit einer Säure ausgewählt sind.

**18.** Zusammensetzung nach Anspruch 17, **dadurch gekennzeichnet, dass** die Kuppler unter 2-Methyl-5-amino-phenol, 5-N-(β-Hydroxyethyl)-amino-2-methyl-phenol, 3-Amino-phenol, 1,3-Dihydroxy-benzol, 1,3-Dihydroxy-2-methyl-benzol, 4-Chlor-1,3-dihydroxy-benzol, 2,4-Diamino-1-(β-hydroxyethyloxy)-benzol, 2-Amino-4-(β-hydroxyethylamino)-1-methoxy-benzol, 1,3-Diaminobenzol, 1,3-Bis(2,4-diaminophenoxy)-propan, Sesamol, 1-Amino-2-methoxy-4,5-methylendioxy-benzol, α-Naphthol, 6-Hydroxyindol, 4-Hydroxyindol, 4-Hydroxy-N-methyl-indol, 6-Hydroxyindolin, 2,6-Dihydroxy-4-methyl-pyridin, 1-H-3-Methyl-pyrazol-5-on, 1-Phenyl-3-methyl-pyrazol-5-on und den Additionssalzen dieser Verbindungen mit einer Säure ausgewählt sind.

**19.** Zusammensetzung nach einem der Ansprüche 16 bis 18, **dadurch gekennzeichnet, dass** der oder die Kuppler 0,0001 bis 10 Gew.-% des Gesamtgewichts der gebrauchsfertigen Farbmittelzusammensetzung ausmachen.

**20.** Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten ausgewählt sind.

**21.** Verfahren zum oxidativen Färben von Keratinfasern und insbesondere menschlichen Keratinfasern, wie Haaren, **dadurch gekennzeichnet, dass** auf die Fasern während einer Zeitspanne, die zur Entwicklung der gewünschten Färbung ausreichend ist, mindestens eine gebrauchsfertige Farbmittelzusammensetzung nach einem der Ansprüche 1 bis 20 aufgebracht wird.

**22.** Verfahren nach Anspruch 21, **dadurch gekennzeichnet, dass** die zur Entwicklung der Färbung auf den Keratinfasern erforderliche Zeitspanne im Bereich von 3 bis 60 min liegt.

**23.** Verfahren nach Anspruch 21 oder 22, **dadurch gekennzeichnet, dass** es einen vorbereitenden Schritt umfasst, der darin besteht, einerseits eine Zusammensetzung (A), die in einem zum Färben geeigneten Medium mindestens eine Oxidationsbase enthält, und andererseits eine Zusammensetzung (B) getrennt voneinander aufzubewahren, die in einem zum Färben geeigneten Medium mindestens ein Enzym vom Typ der ammoniogenen Oxidoreduktasen (2 Elektronen) enthält, wobei die Zusammensetzung (A) und/ oder die Zusammensetzung (B) mindestens einen Donor für das Enzym enthält, und diese bei der Anwendung zu vermischen, bevor das Gemisch auf die Keratinfasern aufgebracht wird.

## Claims

**1.** Ready-to-use composition for the oxidation dyeing of keratin fibres, and in particular human keratin fibres such as the hair, **characterized in that** it comprises, in a medium which is suitable for dyeing:

- at least one oxidation base,
- at least one ammonia-generating 2-electron oxidoreductase-type enzyme,
- and at least one donor for the said enzyme.

**2.** Composition according to Claim 1, **characterized in that** the oxidation bases are chosen from para-phenylenediamines, double bases, para-aminophenols, ortho-aminophenols and heterocyclic oxidation bases.

**3.** Composition according to Claim 2, **characterized in that** the oxidation bases are chosen from para-phenylenediamines, double bases, para-aminophenols and ortho-aminophenols.

**4.** Composition according to Claim 2 or 3, **characterized in that** the para-phenylenediamines are chosen from the compounds of formula (I) below, and the addition salts thereof with an acid:

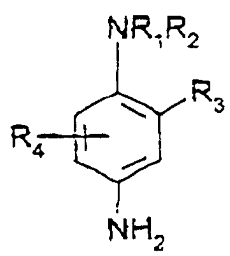

(I)

in which:

- $R_1$ represents a hydrogen atom, a $C_1$-$C_4$ alkyl radical, a $C_1$-$C_4$ monohydroxyalkyl radical, a $C_2$-$C_4$ polyhydroxyalkyl radical, a ($C_1$-$C_4$)alkoxy($C_1$-$C_4$)alkyl radical, a $C_1$-$C_4$ alkyl radical substituted with a nitrogenous group, a phenyl radical or a 4'-aminophenyl radical;
- $R_2$ represents a hydrogen atom, a $C_1$-$C_4$ alkyl radical, a $C_1$-$C_4$ monohydroxyalkyl radical, a $C_2$-$C_4$ polyhydroxyalkyl radical, a ($C_1$-$C_4$)alkoxy($C_1$-$C_4$)alkyl radical or a $C_1$-$C_4$ alkyl radical substituted with a nitrogenous group;
- $R_3$ represents a hydrogen atom, a halogen atom such as a chlorine, bromine, iodine or fluorine atom, a $C_1$-$C_4$ alkyl radical, a $C_1$-$C_4$ monohydroxyalkyl radical, a $C_1$-$C_4$ hydroxyalkoxy radical, an acetylamino($C_1$-$C_4$)-alkoxy radical, a $C_1$-$C_4$ mesylaminoalkoxy radical or a carbamoylamino($C_1$-$C_4$)alkoxy radical,
- $R_4$ represents a hydrogen or halogen atom or a $C_1$-$C_4$ alkyl radical.

**5.** Composition according to Claim 4, **characterized in that** the para-phenylenediamines of formula (I) are chosen from para-phenylenediamine, para-tolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine,

4-N,N-bis(β-hydroxyethyl)amino-2-methylaniline, 4-N,N-bis(β-hydroxyethyl)amino-2-chloroaniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylene-diamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylene-diamine, 2-β-acetylaminoethyloxy-para-phenylenediamine and N-(β-methoxyethyl)-para-phenylenediamine, and the addition salts thereof with an acid.

**6.** Composition according to Claim 2 or 3, **characterized in that** the double bases are chosen from the compounds of formula (II) below, and the addition salts thereof with an acid:

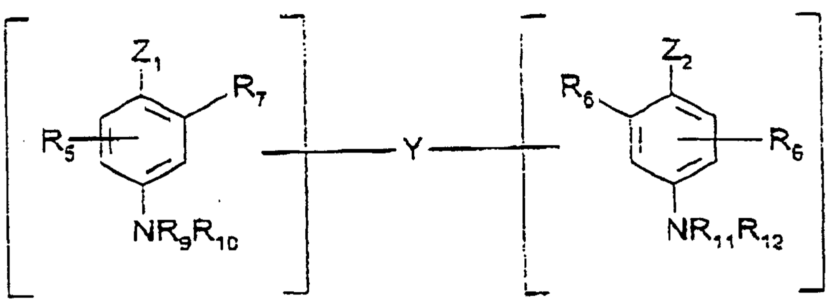

(II)

in which:

- $Z_1$ and $Z_2$, which may be identical or different, represent a hydroxyl or $-NH_2$ radical which may be substituted with a $C_1$-$C_4$ alkyl radical or with a linker arm Y;
- the linker arm Y represents a linear or branched alkylene chain containing from 1 to 14 carbon atoms, which may be interrupted by or terminated with one or more nitrogenous groups and/or one or more hetero atoms such as oxygen, sulphur or nitrogen atoms, and optionally substituted with one or more hydroxyl or $C_1$-$C_6$ alkoxy radicals;
- $R_5$ and $R_6$ represent a hydrogen or halogen atom, a $C_1$-$C_4$ alkyl radical, a $C_1$-$C_4$ monohydroxyalkyl radical, a $C_2$-$C_4$ polyhydroxyalkyl radical, a $C_1$-$C_4$ aminoalkyl radical or a linker arm Y;
- $R_7$, $R_8$, $R_9$, $R_{10}$, $R_{11}$ and $R_{12}$, which may be identical or different, represent a hydrogen atom, a linker arm Y or a $C_1$-$C_4$ alkyl radical;

it being understood that the compounds of formula (II) contain only one linker arm Y per molecule.

**7.** Composition according to Claim 6, **characterized in that** the double bases of formula (II) are chosen from N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)-tetramethylenediamine, N,N'-bis(β-hydroxyethyl)-N,N'-bis (4-aminophenyl)tetra-methylenediamine, N,N'-bis(4-methylaminophenyl)tetra-methylenediamine, N,N'-bis(ethyl) -N,N'-bis(4'-amino-3'-methylphenyl)ethylenediamine and 1,8-bis(2,5-diaminophenoxy)-3,5-dioxaoctane, and the addition salts thereof with an acid.

**8.** Composition according to Claim 2 or 3, **characterized in that** the para-aminophenols are chosen from the compounds of formula (III) below, and the addition salts thereof with an acid:

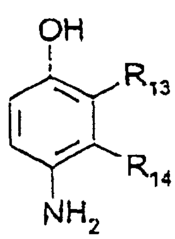

(III)

in which:

- $R_{13}$ represents a hydrogen or halogen atom, a $C_1$-$C_4$ alkyl radical, a $C_1$-$C_4$ monohydroxyalkyl radical, a ($C_1$-$C_4$) alkoxy($C_1$-$C_4$)alkyl radical, a $C_1$-$C_4$ aminoalkyl radical or a hydroxy($C_1$-$C_4$)alkylamino($C_1$-$C_4$)alkyl radical,
- $R_{14}$ represents a hydrogen or halogen atom, a $C_1$-$C_4$ alkyl radical, a $C_1$-$C_4$ monohydroxyalkyl radical, a $C_2$-$C_4$ polyhydroxyalkyl radical, a $C_1$-$C_4$ aminoalkyl radical, a $C_1$-$C_4$ cyanoalkyl radical or a ($C_1$-$C_4$)-alkoxy($C_1$-$C_4$) alkyl radical, it being understood that at least one of the radicals $R_{13}$ or $R_{14}$ represents a hydrogen atom.

**9.** Composition according to Claim 8, **characterized in that** the para-aminophenols of formula (III) are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxy-ethylaminomethyl)phenol and 4-amino-2-fluorophenol, and the addition salts thereof with an acid.

**10.** Composition according to Claim 2 or 3, **characterized in that** the ortho-aminophenols are chosen from 2-aminophenol, 2-amino-5-methylphenol, 2-amino-6-methylphenol and 5-acetamido-2-aminophenol, and the addition salts thereof with an acid.

**11.** Composition according to Claim 2, **characterized in that** the heterocyclic bases are chosen from pyridine derivatives, pyrimidine derivatives and pyrazole derivatives, and the addition salts thereof with an acid.

**12.** Composition according to any one of the preceding claims, **characterized in that** the oxidation base(s). represent (s) from 0.0005 to 12% by weight relative to the total weight of the ready-to-use dye composition.

**13.** Composition according to any one of the preceding claims, **characterized in that** the ammonia-generating 2-electron oxidoreductase-type enzymes are chosen from D-aspartate oxidases, L-amino acid oxidases, D-amino acid oxidases, (mono)amine oxidases containing a flavin, pyridoxamine phosphate oxidases, (di)amine oxidases containing copper, D-glutamate oxidases, ethanolamine oxidases, putrescine oxidases, L-glutamate oxidases, cyclohexylamine oxidases, protein-lysine-6-oxidases, L-lysine oxidases, D-glutamate (D-aspartate) oxidases and L-aspartate oxidases.

**14.** Composition according to any one of the preceding claims, **characterized in that** the ammonia-generating 2-electron oxidoreductase(s) preferably represent(s) from 0.01 to 40% by weight relative to the total weight of the ready-to-use dye composition.

**15.** Composition according to any one of the preceding claims, **characterized in that** the donor(s) represent(s) from 0.01 to 20% by weight relative to the total weight of the ready-to-use dye composition.

**16.** Composition according to any one of the preceding claims, **characterized in that** it contains one or more couplers and/or one or more direct dyes.

**17.** Composition according to Claim 16, **characterized in that** the couplers are chosen from meta-phenylenediamines, meta-aminophenols, meta-diphenols and heterocyclic couplers, and the addition salts thereof with an acid.

**18.** Composition according to Claim 17, **characterized in that** the couplers are chosen from 2-methyl-5-aminophenol, 5-N-(β-hydroxyethyl)amino-2-methylphenol, 3-aminophenol, 1,3-dihydroxybenzene, 1,3-dihydroxy-2-methylbenzene, 4-chloro-1,3-dihydroxy-benzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, sesamol, 1-amino-2-methoxy-4,5-methylenedioxybenzene, α-naphthol, 6-hydroxyindole, 4-hydroxyindole, 4-hydroxy-N-methylindole, 6-hydroxyindoline, 2,6-dihydroxy-4-methylpyridine, 1-H-3-methylpyrazole-5-one and 1-phenyl-3-methylpyrazol-5-one, and the addition salts thereof with an acid.

**19.** Composition according to any one of Claims 16 to 18, **characterized in that** the coupler(s) represent(s) from 0.0001 to 10% by weight relative to the total weight of the ready-to-use dye composition.

**20.** Composition according to any one of the preceding claims, **characterized in that** the addition salts with an acid are chosen from the hydrochlorides, hydrobromides, sulphates, tartrates, lactates and acetates.

**21.** Process for the oxidation dyeing of keratin fibres, and in particular human keratin fibres such as the hair, **characterized in that** at least one ready-to-use dye composition as defined in any one of Claims 1 to 20 is applied to the

said fibres, for a period which is sufficient to develop the desired coloration.

**22.** Process according to Claim 21, **characterized in that** the time required to develop the coloration on the said keratin fibres is between 3 and 60 minutes.

**23.** Process according to Claim 21 or 22, **characterized in that** it includes a first step which consists in separately storing, on the one hand, a composition (A) comprising, in a medium which is suitable for dyeing, at least one oxidation base and, on the other hand, a composition (B) containing, in a medium which is suitable for dyeing, at least one ammonia-generating 2-electron oxidoreductase-type enzyme, the said composition (A) and/or the said composition (B) containing at least one donor for the said enzyme, and then in mixing them together at the time of use, before applying this mixture to the keratin fibres.